## Europäisches Patentamt
## European Patent Office
## Office européen des brevets

(11) Publication number: **0 045 655**
**B1**

(12) **EUROPEAN PATENT SPECIFICATION**

(45) Date of publication of patent specification: **20.03.85**

(51) Int. Cl.⁴: $A\ 61\ K\ 9/00$

(21) Application number: **81303545.8**

(22) Date of filing: **03.08.81**

(54) Solubilization of ivermectin in water.

(30) Priority: **04.08.80 US 174957**

(43) Date of publication of application:
**10.02.82 Bulletin 82/06**

(45) Publication of the grant of the patent:
**20.03.85 Bulletin 85/12**

(84) Designated Contracting States:
**AT BE CH DE FR GB IT LI LU NL SE**

(56) References cited:
**GB-A- 784 659
GB-A- 958 177
GB-A-1 225 979
US-A-4 199 569**

**MERCK INDEX ENCYCLOPEDIA OF
CHEMICALS AND DRUGS, Ninth edition,
Merck & Co., 1976, Rahway;**

(73) Proprietor: **MERCK & CO. INC.
126, East Lincoln Avenue P.O. Box 2000
Rahway New Jersey 07065 (US)**

(72) Inventor: **Lo, Pak-Kan Albert
17 Lombardi Street
Edison New Jersey 08817 (US)**
Inventor: **Williams, James B.
4 Coventry Drive
Freehold New Jersey 07728 (US)**

(74) Representative: **Crampton, Keith John Allen
et al
D YOUNG & CO 10 Staple Inn
London WC1V 7RD (GB)**

Courier Press, Leamington Spa, England.

## Description

Ivermectin is a new and very potent antiparasitic, and particularly anthelmintic, agent that is useful against a broad spectrum of endo-parasites and ectoparasites in mammals as well as having agricultural uses against parasites found in and on crops and in soil. Ivermectin is disclosed in U.S. Patent 4,166,569, issued 22 April, 1980 to Chabala and Fisher. Ivermectin is a mixture, in the ratio of approximately 80:20 of 22,23-dihydro C-076 Bla and Blb. In administering Ivermectin to animals it is most convenient for parenteral formulations to use an aqueous solution. Non-aqueous solutions tend to cause irritation and tissue damage at the injection site; precipitate the active ingredient at the injection site; have higher viscosity and poorer syringability; and generally have a higher cost. Aqueous liquid formulations for oral use are also preferred over non-aqueous formulations because non-aqueous solvents tend to have an unacceptable taste.

Thus, it is desirable to prepare an aqueous liquid formulation of Ivermectin. However, Ivermectin has very poor solubility in water, at a level of about 0.005 mg per ml at room temperature.

Ivermectin can be solubilized using surface-active agents as solubilizers. This results in the formation of micelles, or minute colloidal particles which surround the Ivermectin molecule, isolating it from the water, but forming a clear solution in the water. Such a solution does contain sufficient active ingredient in order to prepare liquid formulations for oral or parenteral use. However, it was discovered that such micelle formulations were unstable and the Ivermectin degraded at such a rate as to render the shelf life inadequate for a commercial preparation.

It was unexpectedly discovered during the investigation of this instability that the use of certain cosolvents and/or substrates, would reduce the instability and result in an aqueous liquid solution which is suitable for parenteral or oral administration, and which had adequate shelf life such that a viable commercial preparation was afforded.

The present invention concerns the solubilization and stabilization of Ivermectin using surface-active agents to dissolve the Ivermectin, and certain co-solvents and substrates to stabilize the thus formed micelle solution, and this invention provides an aqueous formulation comprising Ivermectin in a solution of a non-ionic or an anionic surface-active agent and water, characterized in that the formulation is stabilized by the inclusion of one or more water-miscible organic cosolvents suitable for patenteral or oral administration, the cosolvent(s) being glycerol formal, propylene glycol, glycerine, and/or polyethylene glycol; and/or one or more substrates also suitable for parenteral or oral administration, the substrate(s) being benzyl alcohol, lidocaine, a paraben, and/or choline. The stabilized solution can be used to prepare patenteral and oral formulations.

The cosolvent and the substrate used in the formulations of the present invention individually reduce the instability of the Ivermectin solution: however, the combination of the cosolvent and the substrate is surprisingly found to increase the stability of the solution even further.

The aqueous Ivermectin solution is initially formed by dissolving the Ivermectin in a pharmacologically acceptable surface-active agent. A different surface-active agent will be used depending upon whether the final formulation is to be for parenteral or oral use.

For patenteral use a pharmacologically acceptable non-ionic surface-active agent will be used. Examples of such non-ionic surface-active agents are polyoxyethylated vegetable oils, polyoxyethylene sorbitan monoisostearate, polyoxyethylene sorbitan monostearate and polyoxyethylenesorbitan monooleate (also known as polysorbate 80 or under the trade mark Tween 80). The preferred surface-active agent is a polysorbate 80.

For oral use, a pharmacologically acceptable non-ionic surfactant or an anionic surface-active agent will be used. The non-ionic surface-active agents mentioned for the parenteral formulation may also be used for the oral formulation, and again polysorbate 80 is the preferred non-ionic surface-active agent. An example of an anionic surface-active agent is dioctylsodium sulfosuccinate (also known as Aerosol OT), which is the preferred anionic surface-active agent.

The aqueous solution of Ivermectin and the surface-active agent is prepared by dissolving the Ivermectin in the surface-active agent such that the surface-active agent will constitute from 4 to 25% w/v of the final solution. The Ivermectin is present in different amounts for parenteral and oral use. For parenteral formulations the Ivermectin is present at from 0.1 to 7.5% w/v and for oral formulations the Ivermectin is present in from 0.01 to 2.0% w/v. Water may then be added to the solution of surface-active agent to form a clear solution.

The preferred cosolvent for parenteral administration is glycerol formal and for oral administration is propylene glycol. The co-solvents are added to the final formulation to the extent of 10 to 40% v/v of the final formulation.

Of the substrates used to stabilize the formulation, either alone or in combination with the cosolvent, benzyl alcohol and lidocaine are preferred and both have been used in a single formulation with acceptable results. The substrates are present in the final formulations at a concentration of from 1 to 5% w/v. Benzyl alcohol is specifically present at 1 to 5% v/v and lidocaine is present at 1 to 4% w/v.

The preferred process for preparing the formulation is to combine the Ivermectin in a mixture of the surface active agent, the cosolvent and the substrate. At this time also buffering agents and other adjuvants which assist in the final formulation may be added. Water is then added to the desired volume, or almost the desired volume, and the pH adjusted, if necessary, to a range of 6.0 and 6.5 for optimum stability. The final volume is adjusted to the desired amount and the solution sterilized by autoclaving or membrane filtration.

The stability of the Ivermectin aqueous solution is thus greatly improved through the use of the above-described cosolvents and substrates. Without such cosolvents and substrates, the solution of Ivermectin formed by combining the drug in a surfactant and adding water is observed to have a 50% stability per month at room temperature. That is, 50% of the Ivermectin is lost after only one month. By combining a cosolvent or a substrate with the surfactant, the stability is seen to dramatically increase to about 10% in 2 to 3 months; or about 5% loss of Ivermectin activity per month. When both the cosolvent and the substrate are used in the surfactant formulation the stability of the resultant aqueous formulation is seen to even more dramatically increase its stability to less than 5% in 2 to 3 years.

The reason behind this dramatic and unexpected stabilizing effects resulting from the use of the cosolvent and the substrate are not completely understood. While this theory is not to be taken as binding, it appears that in the initial micelle formation with the Ivermectin and the substrate, water is still able to penetrate the micelle or otherwise contact the Ivermectin, even though it is surrounded by the surface-active agent. The cosolvent and the substrate apparently displace the water of hydration of the micelle and further isolate the Ivermectin from the water that contacts the outside surface of the micelle, thus reducing the reaction of the water upon the Ivermectin and increasing the stability of the resultant solution.

The resultant solution thus avoids the disadvantages of non-aqueous formulations while retaining the required attributes of a parenteral or oral formulation. The solution is stable, both chemically and physically; it is low in viscosity, therefore its syringability is excellent; it does not cause any irritation or tissue damage at the injection site; its taste is not objectionable upon oral administration; the solution is totally dilutable with water with precipitating the Ivermectin; the Ivermectin is rapidly absorbed; and the solution is produced at low cost.

The following illustrative non-limiting examples of aqueous formulations in accordance with the present invention are provided in order that the invention may be more fully understood.

## Example 1
Ivermectin Injectable Solution (10 mg/mL

Formula

| | |
|---|---|
| Ivermectin | 1.0% w/v |
| Polysorbate 80 | 8% w/v |
| Glycerol Formal | 20% w/v |
| Lidocaine | 2% w/v |
| Benzyl Alcohol | 1% v/v |
| Water for Injection | q.s. 100% v/v |

pH adjusted to 6.2 using 1N HCl

Procedure
1. Dissolve Ivermectin and lidocaine in Polysorbate 80, glycerol formal, and benzyl alcohol.
2. Add water for injection equal to 80% of final volume.
3. Adjust pH of the solution to 6.2 using 1N HCl.
4. Adjust the solution to volume with water for injection.
5. Sterilize by autoclave or membrane filtration and package aseptically.

## Example 2
Ivermectin Injectable Solution (20 mg/mL

Formula

| | |
|---|---|
| Ivermectin | 2.0% w/v |
| Polysorbate 80 | 12% w/v |
| Glycerol Formal | 25% v/v |
| Benzyl Alcohol | 3% v/v |
| Sodium Phosphate Dibasic—Anhydrous | 0.1% w/v |
| Sodium Phosphate Monobasic—Monohydrate | 0.9 w/v |
| Water for Injection | q.s. 100% w/v |

Procedure
1. Dissolve Ivermectin in Polysorbate 80, glycerol formal, and benzyl alcohol.
2. Disperse the buffer salts into the solution.
3. Add water for injection and agitate until a clear solution is obtained.
4. Adjust the solution to volume with water for injection.
5. Sterilize by autoclave or membrane filtration and package aseptically.

### Example 3
Ivermectin Oral Solution (0.8 mg/mL

| Formula | |
|---|---|
| Ivermectin | 0.08% w/v |
| Polysorbate 80 | 8.0% w/v |
| Propylene Glycol | 20% v/v |
| Benzyl Alcohol | 3% v/v |
| Sodium Phosphate Dibasic—Anhydrous | 0.1% w/v |
| Sodium Phosphate Monobasic—Monohydrate | 0.9% w/v |
| Water, Purified | q.s. 100% w/v |

Procedure

1. Dissolve Ivermectin in Polysorbate 80, propylene glycol, and benzyl alcohol.

2. Disperse the buffer salts into the solution.

3. Add purified water and agitate until a clear solution is obtained.

4. Adjust the solution to volume with purified water and package.

## Claims

1. An aqueous formulation comprising Ivermectin in a solution of a non-ionic or an anionic surface-active agent and water, characterized in that the formulation is stabilized by the inclusion of one or more water-miscible organic co-solvents suitable for parenteral or oral administration, the cosolvent(s) being glycerol formal, propylene glycol, glycerine, and/or polyethylene glycol; and/or one or more substrates also suitable for parenteral or oral administration, the substrate(s) being benzyl alcohol, lidocaine, a paraben, and/or choline.

2. A formulation as claimed in Claim 1 that contains from 0.1 to 7.5% w/v of Ivermectin for parenteral administration or from 0.01 to 2.0% w/v of Ivermectin for oral administration; from 4 to 25% w/v of the surface-active agent; from 10 to 40% v/v of the cosolvent; and from 1 to 5% w/v of the substrate.

3. A formulation as claimed in Claim 1 or 2, in which the surface-active agent is polyoxyethylated vegetable oil, polyoxyethylene sorbitan monoisostearate, polyoxyethylene sorbitan monostearate, polysorbate 80 or dioctylsodium sulfosuccinate.

4. A formulation as claimed in Claim 1, in which a cosolvent is present but no substrate is included.

5. A formulation as claimed in Claim 4 containing from 0.1 to 7.5% w/v of Ivermectin for parenteral administration or from 0.01 to 2.0% w/v of Ivermectin for oral administration, from 4 to 25% w/v of a polyoxyethylated vegetable oil, polyoxyethylene sorbitan monoisostearate, polyoxyethylene sorbitan monostearate or polysorbate 80, and from 10 to 40% v/v of glycerol formal, propylene glycol, glycerine or polyethylene glycol.

6. A formulation as claimed in Claim 1, in which a substrate is present but no cosolvent is included.

7. A formulation as claimed in Claim 6 comprising from 0.1 to 7.5% w/v of Ivermectin for parenteral administration or from 0.01 to 2.0% w/v of Ivermectin for oral administration; from 4 to 25% w/v of a polyoxyethylated vegetable oil, polyoxyethylene sorbitan monoisostearate, polyoxyethylene sorbitan monostearate or polysorbate 80; and from 1 to 5% w/v of one or more of benzyl alcohol, lidocaine, a paraben, or choline.

8. A process for preparing a stabilized aqueous formulation containing Ivermectin, which comprises dissolving Ivermectin in a non-ionic or an anionic surface-active agent containing one or more cosolvents as defined in Claim 1 and/or one or more substrates as defined in Claim 1; adding water to the thus prepared solution to make up the desired volume and adjusting the pH if necessary.

9. A process as claimed in Claim 8, in which the final solution has a pH adjusted to be in the range 6 to 6.5 and is a solution as claimed in Claim 2 or 3.

10. A process as claimed in Claim 9, in which the surface-active agent is polysorbate 80, the cosolvent is glycerol formal or propylene glycol and the substrate is one or both of benzyl alcohol or lidocaine.

## Revendications

1. Une composition aqueuse comprenant de l'Ivermectine dans une solution d'un agent tensio-actif non ionique ou anionique et d'eau, la composition étant caracterisée en ce qu'elle est stabilisée par incorporation d'un ou plusieurs cosolvants organiques miscibles à l'eau appropriés à l'administration parentérale ou orale, le(s) cosolvant(s) étant le glycérol-formal, le propylèneglycol, la glycérine et/ou le polyéthylèneglycol; et/ou un ou plusieurs substrats convenant également à l'administration parentérale ou orale, le(s) substrat(s) étant l'alcool benzylique, la lidocaïne, un p-hydroxy-benzoate et/ou la choline.

2. Une composition comme revendiquée dans la revendication 1 que contient de 0,1 à 7,5% p/v d'Ivermectine pour l'administration parentérale ou de 0,01 à 2,0% p/v d'Iver-mectine pour l'administration orale; de 4 à 25% p/v de l'agent tensio-actif; de 10 à 40% v/v du cosolvant; et de 1 à 5% p/v du substrat.

3. Une composition comme revendiquée dans la revendication 1 ou 2, dans laquelle l'agent tensio-actif est une huile végétale poly-oxyéthylée, le polyoxyéthylène-monoisostéar-ate de sorbitanne, le polyoxyéthylène-monostéarate de sorbitanne, le polysorbate 80 ou le dioctylsulfosuccinate de sodium.

4. Une composition comme revendiquée dans la revendication 1, dans laquelle un cosolvant est présent mais qui ne contient pas de substrat.

5. Une composition comme revendiquée dans la revendication 4, contenant de 0,1 à 7,5% p/v d'Ivermectine pour l'administration parentérale ou de 0,01 à 2,0% p/v d'Ivermectine pour l'administration orale, de 4 à 25% p/v d'une huile végétale polyoxyéthylée, de polyoxyéthylène-monoisostéarate de sorbitanne, de polyoxyéthylène-monostéarate de sorbitanne, ou de polysorbate 80; et de 10 à 40% v/v de glycérol-formal; de propylèneglycol, de glycérine ou de polyéthylèneglycol.

6. Une composition comme revendiquée dans la revendication 1, dans laquelle un substrat est présent mais qui ne contient pas de cosolvant.

7. Une composition comme revendiquée dans la revendication 6 comprenant de 0,1 à 7,5% p/v d'Ivermectine pour l'administration parentérale ou de 0,01 à 2,0% p/v d'Iver-mectine pour l'administration orale; de 4 à 25% p/v d'une huile végétale polyoxyéthylée, de polyoxyéthylène-monoisostéarate de sorbitanne, de polyoxyéthylene-monostéarate de sorbitanne ou de polysorbate 80; et de 1 à 5% p/v d'un ou plusieurs composés choisis parmi l'alcool benzylique, la lidocaïne, un p-hydroxy-benzoate ou la choline.

8. Un procédé pour préparer une composition aqueuse stabilisée contenant de l'Iver-mectine quie comprend la dissolution de l'Iver-mectine dans uns agent tensio-actif non ionique ou anionique contenant un ou plusieurs cosolvants comme défini dans la revendication 1 et/ou un ou plusieurs substrats comme défini dans la revendication 1; l'addition d'eau à la solution ainsi préparée pour obtenir le volume désiré et au besoin l'ajustement du pH.

9. Un procédé comme revendiqué dans la revendication 8, dans lequel la solution finale a un pH adjusté pour qu'il soit dans la gamme de 6 à 6,5 et est une solution comme revendiqué dans la revendication 2 ou 3.

10. Un procédé comme revendiqué dans la revendication 9, dans lequel l'agent tensio-actif est le polysorbate 80, le cosolvant est le glycérol-formal ou le propylèneglycol et le substrat est l'alcool benzylique et/ou la lidocaïne.

## Patentansprüche

1. Eine wässerige Formulierung, die Ivermectin in einer Lösung eines nichtionischen oder einer anionischen oberflächenaktiven Mittels und Wasser enthält, dadurch gekennzeichnet, daß die Formulierung durch die Einverleibung eines oder mehrerer, für parenterale oder orale Verabreichung geeigneter(er), wassermischbaren(er), organischen(er) Colösungsmittels (Colösungsmittel), wobei das Colösungsmittel bzw. die Colösungsmittel Glycerinformal, Propylenglykol, Glycerin und/oder Polyethylenglykol ist bzw. sind; und/oder eines oder mehrerer, gleichfalls für parenterale oder orale Verabreichung geeigneten(er) Substrates bzw. Substrate, wobei das Substrat bzw. die Substrate Benzylalkohol, Lidocain, ein Paraben und/oder Cholin ist bzw. sind, stabilisiert ist.

2. Eine Formulierung, wie in Anspruch 1 beansprucht, die für parenterale Verabreichung 0,1 bis 7,5% (Gew./Vol.) Ivermectin oder für orale Verabreichung 0,01 bis 2,0% (Gew./Vol.) Ivermectin; 4 bis 25% (Gew./Vol.) des oberflächenaktiven Mittels; 10 bis 40% (Vol./Vol.) des Colösungsmittels; und 1 bis 5% (Gew./Vol.) des Substrates enthält.

3. Eine Formuliering, wie in Anspruch 1 oder 2 beansprucht, in welcher das oberflächenaktive Mittel polyoxyethyliertes Pflanzenöl, Polyoxyethylensorbitanmonoisostearat, Polyoxyethylensorbitanmonostearat, Polysorbat 80 oder Dioctylnatriumsulfosuccinat ist.

4. Eine Formuliering, wie in Anspruch 1 beansprucht, in welcher ein Colösungsmittel, vorliegt, aber kein Substrat einverleibt ist.

5. Eine Formulierung, wie in Anspruch 4 be-ansprucht, enthaltend für parenterale Verab-reichung 0,1 bis 7,5% (Gew./Vol.) Ivermectin oder für orale Verabreichung 0,01 bis 2,0% (Gew./Vol.) Ivermectin, 4 bis 25% (Gew./Vol.) eines polyoxyethylierten Pflanzenöles, Polyoxy-ethylensorbitanmonoisostearat, Polyoxyethylen-sorbitanmonostearat oder Polysorbat 80; und 10 bis 40% (Vol./Vol.) Glycerinformal, Propylenglykol, Glycerin oder Polyethylenglykol.

6. Eine Formuliering, wie in Anspruch 1 beansprucht, in welcher ein Substrat vorliegt, aber kein Colösungsmittel einverleibt ist.

7. Eine Formulierung, wie in Anspruch 6 be-ansprucht, enthaltend für parenterale Verab-reichung 0,1 bis 7,5% (Gew./Vol.) Ivermectin oder für orale Verabreichung 0,01 bis 2,0% (Gew./Vol.) Ivermectin; 4 bis 25% (Gew./Vol.) eines polyoxyethylierten Pflanzenöls, Polyoxy-ethylensorbitanmonoisostearat, Polyoxyethyl-ensorbitanmonostearat oder Polysorbat 80; und 1 bis 5% (Gew./Vol.) Benzylalkohol, Lidocain, eines Parabens oder Cholin, einzeln oder zu mehreren.

8. Ein Verfahren zur Herstellung einer stabilisierten, wässerigen Formulierung, die Ivermectin enthält, umfassend das Auflösen von Ivermectin in einem nichtionischen oder einem anionischen oberflächenaktiven Mittel, das eines oder mehrere Colösungsmittel, wie in Anspruch 1 definiert, und/oder ein Substrat oder mehrere Substrate, wie in Anspruch 1 definiert, enthält; die Zugabe von Wasser zu der so hergestellten Lösung zur Einstellung des gewünschten Volumens und erforderlichenfalls die Einstellung des pH.

9. Ein Verfahren, wie in Anspruch 8 beansprucht, in welchem die Endlösung einen pH aufweist, dar auf einen Wert im Bereich von 6 bis 6,5 eingestellt ist, und eine Lösung darstellt, die in Anspruch 2 oder 3 beansprucht ist.

10. Ein Verfahren, wie in Anspruch 9 beansprucht, in welchem das oberflächenaktive Mittel Polysorbat 80 ist, das Colösungsmittel Glycerinformal oder Propylenglykol ist und das Substrat Benzylalkohol und/oder Lidocain ist.